(19) **Europäisches Patentamt**
**European Patent Office**
**Office européen des brevets**

(11) **EP 1 679 300 B1**

(12) **EUROPEAN PATENT SPECIFICATION**

(45) Date of publication and mention
of the grant of the patent:
**02.05.2007 Bulletin 2007/18**

(51) Int Cl.:
*C07C 69/33* *(2006.01)*

(21) Application number: **05025879.7**

(22) Date of filing: **28.11.2005**

(54) **Process for producing polyglycerol fatty acid esters**

Verfahren zur Herstellung von Polyglycerinfettsäureester

Procedé de production d'esters d'acide gras polyglyceriques

(84) Designated Contracting States:
**AT BE BG CH CY CZ DE DK EE ES FI FR GB GR HU IE IS IT LI LT LU LV MC NL PL PT RO SE SI SK TR**

(30) Priority: **07.01.2005 JP 2005003208**
**11.07.2005 JP 2005202253**

(43) Date of publication of application:
**12.07.2006 Bulletin 2006/28**

(73) Proprietor: **RIKEN VITAMIN CO., LTD.**
**Chiyoda-ku,**
**Tokyo 101-8370 (JP)**

(72) Inventors:
• **Nagahiro, Shinya c/o RIKEN VITAMIN CO.,LTD.**
**Hirakata-shi**
**Osaka 573-0065 (JP)**

• **Kamiryou, Eiji c/o RIKEN VITAMIN CO.,LTD.**
**Hirakata-shi**
**Osaka 573-0065 (JP)**

(74) Representative: **Teipel, Stephan et al**
**Lederer & Keller**
**Patentanwälte**
**Prinzregentenstrasse 16**
**80538 München (DE)**

(56) References cited:
**US-A- 5 247 114          US-A- 5 466 719**

• **PATENT ABSTRACTS OF JAPAN vol. 018, no. 268 (C-1202), 23 May 1994 (1994-05-23) & JP 06 041007 A (MITSUBISHI KASEI CORP), 15 February 1994 (1994-02-15)**
• **PATENT ABSTRACTS OF JAPAN vol. 012, no. 230 (C-508), 29 June 1988 (1988-06-29) & JP 63 023837 A (SAKAMOTO YAKUHIN KOGYO KK), 1 February 1988 (1988-02-01)**

**Description**

BACKGROUND OF THE INVENTION

1. FIELD OF THE INVENTION

The present invention relates to a process for producing a polyglycerol fatty acid ester.

2. DESCRIPTION OF RELATED ART

Polyglycerol fatty acid esters are a surfactant which is highly safe for human body, and have been widely used as food additives and for the production of emulsions or solubilizers in the field of cosmetics and pharmaceuticals. Polyglycerol fatty acid esters having a wide range of HLB value from hydrophilicity to lipophilicity have a characteristic in that they can be obtained by selecting the average polymerization degree of hydrophilic polyglycerols, the kind of lipophilic fatty acids as lipophilic group, and the esterification degree.

With regard to a process for producing polyglycerol fatty acid esters, there are known a process by a direct esterification reaction between polyglycerol and a fatty acid (or a fatty acid chloride, etc.), a process by a transesterification reaction of polyglycerol with a fat and oil (or a fatty acid methyl ester, etc.), and a process by an addition polymerization reaction of a glycidol with a fatty acid. Currently, an industrial process adopted domestically and internationally is a process by a direct esterification between a polyglycerol and a fatty acid.

In the direct esterification reaction of a polyglycerol with a fatty acid, a reaction mixture enriched with monoesters is obtained by the reaction wherein not more than 1 mol of a fatty acid is used relative to 1 mol of a polyglycerol. However, it is inevitable that considerable amounts of unreacted polyglycerol remain in such a reaction mixture of low esterification degree. Since polyglycerols have a very high boiling point, it is not able to efficiently remove polyglycerols by means of a reduced pressure distillation, etc. Therefore, polyglycerol fatty acid esters having a high HLB value are put on the market usually as a commercial product containing unreacted polyglycerol.

Polyglycerols are one of polyhydric alcohols containing ether bonds in the molecule and have no function as a surfactant. Therefore, with regard to polyglycerol fatty acid esters containing unreacted polyglycerol, the effect as a surfactant per unit weight is decreased in proportion to the unreacted polyglycerol content. In addition, the product containing large amounts of the unreacted polyglycerols has a defect that it is very sticky and difficult for its handling.

Accordingly, various methods for enhancing the content of polyglycerol fatty acid esters have been studied by separating the polyglycerol fatty acid ester and the unreacted polyglycerol from the reaction mixture after the esterification. Such known methods include, for example, a method where the reaction mixture is allowed to stand at 90°C for 1 hour and the upper layer is separated to obtain a polyglycerol fatty acid ester (see Patent Literature 1: JP-1983-185537A, Synthetic Example 1); a method for producing a polyglycerol fatty acid ester, which comprises reacting a polyglycerol with a fatty acid at 140 to 220°C, cooling down the reaction mixture to 60 to 115°C, optionally removing the precipitated polyglycerol component, adding an organic solvent to the reaction mixture containing unreacted polyglycerol component at 0 to 100°C, extracting the unreacted polyglycerol component through at least one extraction step with water which is thereafter added, and distilling the separated organic phase to separate a mixture of polyglycerol fatty acid esters from the organic solvent used and the residual water contained therein (see Patent Literature 2: JP-1993-78279A, on page 3, left column, lines 37-47); a method for producing a polyglycerol fatty acid ester with high HLB, which comprises removing unreacted polyglycerol from a reaction product obtained by esterification reaction of not more than 1.0 mol of a fatty acid with 1.0 mol of a polyglycerol, through liquid/liquid separation using a solvent, and optionally evaporating off the solvent (see Patent Literature 3: JP-1988-23837A); a method for producing a highly purified polyglycerol fatty acid ester, which comprises bringing a solution of esterification product of a polyglycerol with a fatty acid, into contact with alkylsilylated silica gel to adsorb the reaction product, removing firstly unreacted polyglycerols by extraction with a solution containing 15 to 90% by volume of a lower alcohol, and then eluting the obtained polyglycerol fatty acid ester with an organic solvent capable of dissolving the ester (see Patent Literature 4: JP-1991-81252A); and a method for producing a highly purified polyglycerol fatty acid ester, which comprises reacting a polyglycerol with a fatty acid or a fatty acid ester, and removing unreacted polyglycerol from an esterification product by extraction using a combination of a water-soluble organic solvent and water or an aqueous solution containing a salting-out agent (see Patent Literature 5: JP-1994-41007A).

However, sufficient separation cannot be attained by allowing the reaction mixture for standing only. Also, production cost is increased, and furthermore problems of safety and health have occurred in the method using an organic solvent. Accordingly, these methods are not thought to be preferable. Therefore, there has been desired a production development for producing a polyglycerol fatty acid ester, which enables the unreacted polyglycerol to be separated easily and efficiently.

SUMMARY OF THE INVENTION

An object of the present invention is to provide a method for producing a polyglycerol fatty acid ester, which comprises reducing an unreacted polyglycerol as much as possible from a reaction mixture obtained through a direct esterification reaction between a polyglycerol and a fatty acid in an industrially advantageous manner.

As a result of intensive studies about the above problem, the present inventors have found that the above problem can be solved by adding glycerol to a reaction mixture obtained through a direct esterification reaction between a polyglycerol and a fatty acid, and separating and removing a glycerol phase containing unreacted polyglycerol. The present invention has been made based on these findings.

That is to say, the present invention relates to:

a process for producing a polyglycerol fatty acid ester, which comprises adding glycerol to a reaction mixture obtained by a direct esterification reaction between polyglycerol and a fatty acid at the temperature from 60°C to less than 180°C, and separating and removing the glycerol phase containing unreacted polyglycerols.

According to the present invention, a polyglycerol fatty acid ester containing a low amount of unreacted polyglycerol can be produced simply and efficiently without requiring an organic solvent. Since the polyglycerol fatty acid ester obtained by the process according to the present invention contains a small amount of the unreacted polyglycerol, an effect as a surfactant per unit weight is improved. In addition, the polyglycerol fatty acid ester whose constituent fatty acid is a stearic acid, etc. can be pulverized, and the obtained powder has no sticky property and is easy to handle.

DETAILED DESCRIPTION OF THE INVENTION

The polyglycerol used in the present invention is a mixture of polyglycerols having various degrees of polycondensation obtained by usually adding a small amount of an acid or an alkali as a catalyst, and polycondensing glycerol at a temperature of, for example, not less than about 180°C under an optional inert gas atmosphere such as nitrogen or carbon dioxide. In addition, the polyglycerol may be obtained with use of a glycidol or an epichlorohydrin as a starting material. After the reaction, if required, treatments such as neutralization, desalination, and discoloration may be performed. Examples of said polyglycerol include a polyglycerol composition having an average degree of polycondensation of about 2 to 20 of glycerol, preferably about 2 to 10, such as diglycerol (average degree of polycondensation is 2), triglycerol (average degree of polycondensation is 3), tetraglycerol (average degree of polycondensation is 4), hexaglycerol (average degree of polycondensation is 6), octaglycerol (average degree of polycondensation is 8), and decaglycerol (average degree of polycondensation is 10).

A highly purified polyglycerol in which single component is highly concentrated is preferably used in the present invention, wherein the above polyglycerol mixture having various degrees of polycondensation is purified by the method known per se such as distillation and column chromatography, and the like. Such examples include, for example, a diglycerol composition wherein the content of diglycerol composed of two glycerol molecules is not less than about 50% by mass, preferably not less than about 70% by mass, more preferably not less than about 90% by mass, and a triglycerol composition wherein the content of triglycerol composed of three glycerol molecules is not less than about 50% by mass, preferably not less than about 70% by mass, more preferably not less than about 90% by mass.

The fatty acid used in the present invention is not particularly limited and, for example, there are exemplified straight- or branched-chain, and saturated or unsaturated fatty acids having 6 to 24 carbon atoms. Specifically, such fatty acids include caproic acid, caprylic acid, capric acid, lauric acid, myristic acid, palmitic acid, stearic acid, behenic acid, lignoceric acid, oleic acid, elaidic acid, linoleic acid, linolenic acid, erucic acid, isostearic acid, 2-ethylhexyl acid, and condensed ricinoleic acid, and the like. These fatty acids can be used in combination of one or two or more of them.

In the present invention, a feed amount of a fatty acid relative to a polyglycerol, though it varies depending on the desired esterification degree and is not uniform, is preferably within the range of about 0.1 to 1 mol relative to 1 mol of a polyglycerol. When the feed amount of a fatty acid relative to a polyglycerol is too small, the product amount of a polyglycerol fatty acid ester itself becomes smaller, which is not advantageous from economical standpoint.

In the present invention, the esterification reaction of a polyglycerol with a fatty acid may be performed without a catalyst, or may be performed with an acid catalyst or an alkali catalyst. However, the reaction is preferably performed in the presence of an alkali catalyst. Examples of such acid catalysts include, for example, concentrated sulfuric acid and p-toluene sulfonic acid, and the like. Examples of such alkali catalysts include, for example, potassium hydroxide, sodium hydroxide, potassium carbonate, and sodium carbonate, and the like. The amount of the alkali catalyst used is about $5 \times 10^{-7}$ to 1-fold mole, preferably about $5 \times 10^{-6}$ to 0.1-fold mole to the polyglycerol.

The above esterification reaction is performed by supplying a polyglycerol, a fatty acid, and a catalyst to a conventional reaction vessel comprising, for example, an agitator, a jacket for heating, a baffle, an inert gas blowing tube, a temperature gauge and a water separator with a cooler; stirring the mixture; and heating the mixture at a predetermined temperature for a fixed time while removing water generated by esterification reaction outside the reaction system under atmosphere of an optional inert gas such as nitrogen and carbon dioxide. The reaction temperature is usually in a range of about 180 to 260°C, preferably about 200 to 250°C. The reaction pressure condition is under reduced pressure or normal pressure. The reaction time is about 0.5 to 15 hours, preferably about 1 to 3 hours. The end point of the reaction is usually determined by an acid value of the reaction mixture when the measured acid value is about not more than 12.

After the end of esterification reaction, the catalysts remaining in the reaction mixture are neutralized when the catalysts are used. In that case, when the esterification reaction temperature is not less than 200°C, it is preferable that the neutralization is performed after the reaction solution is cooled down to about 180 to 200°C. When the reaction temperature is not more than 200°C, the reaction solution may be neutralized as it is. The neutralization of catalysts is performed by the following procedure. For example, when sodium hydroxide is used as an alkali catalyst and neutralized with phosphoric acid (85% by mass), to the reaction mixture is added phosphoric acid (85% by mass) in an amount*) of not less than an amount obtained by dividing phosphoric acid amount calculated according to the following neutralization equation (1) by 0.85, preferably in an amount of about 2 to 3 times the amount of phosphoric acid (85% by mass) obtained by dividing phosphoric acid amount calculated according to the following neutralization equation (1) by 0.85, and the resulting mixture is neutralized well. After the neutralization, the reaction mixture is allowed to stand at the neutralization temperature preferably for not less than about 0.5 hour, more preferably for about 1 to 10 hours. If the unreacted polyglycerol is separated into the lower phase, it should be removed.

*) When the amount of sodium hydroxide used is 1.0 g, the amount becomes to be about 0.96 g.

$$[\text{Chemical reaction equation 1}] \; 3NaOH + H_3PO_4 \rightarrow Na_3PO_4 + 3H_2O \qquad (1)$$

Subsequently, the above reaction mixture is kept at the temperature from about 60°C to less than 180°C, preferably at the temperature from about 120°C to less than 180°C, more preferably at about 130 to 150°C, while cooling down if required. And then, to the reaction mixture is added glycerol which is about 0.5 to 10 times, more preferably about 0.5 to 5 times, the total mass of a polyglycerol and a fatty acid at the reaction feed. After the reaction mixture and the glycerol are mixed well, the mixture is allowed to stand for not less than about 0.5 hour, preferably for about 1 to 10 hours at the same temperature, and the lower phase (the glycerol phase containing the unreacted polyglycerol) of separated two phases is withdrawn or centrifuged to remove the glycerol phase containing the unreacted polyglycerol. When the amount of glycerol added is small, it is insufficient to remove the unreacted polyglycerol. When the amount of glycerol added is too large, it takes a long time to separate and remove the unreacted polyglycerol, resulting in undesirable decrease of productivity.

The polyglycerol fatty acid ester composition obtained by the above treatment is preferably distilled under reduced pressure to remove the remaining glycerol, and if required, treatments such as desalting, discoloration, and filtration are performed, thereby to obtain a polyglycerol fatty acid ester wherein the amount of the unreacted polyglycerol is reduced as much as possible.

The glycerol containing the unreacted polyglycerol which was removed by the above operation is recovered, and treatment such as discoloration and the like is optionally performed. The recovered glycerol can be reused as a starting material for polyglycerol production.

EXAMPLES

The following examples are provided to illustrate the present invention more specifically, however, the present invention is not limited to the following examples.

Working Example 1

20 kg of glycerol was fed to a reaction vessel equipped with an agitator, a temperature gauge, a gas blowing tube, and a water separator. 100 mL of 20 w/v% aqueous sodium hydroxide solution as a catalyst was added thereto. Then, a polycondensation reaction of glycerol was performed at 250°C for 4 hours in a nitrogen gas stream.

The resultant reaction product was cooled down to 90°C and about 20 g of phosphoric acid was added thereto for neutralization, and then filtered. The filtrate was distilled under reduced pressure at 160°C and 400 Pa to remove the glycerol. Subsequently, molecular distillation was performed under high vacuum conditions of 200°C and 20 Pa to obtain about 3.7 kg of a distillate containing 2% by mass of glycerol, 91% by mass of diglycerol, and 7% by mass of triglycerol. The distillate was subjected to discoloration by addition of 1% by mass of an activated carbon relative to the distillate, under reduced pressure, followed by filtration. The resultant diglycerol composition had a hydroxy value of about 1350 and the average degree of polycondensation was about 2.0.

To 1 L of a four necked flask comprising an agitator, a temperature gauge, a gas blowing tube, and a water separator were fed 166 g (about 1.0 mol) of the above diglycerol composition and 226 g (about 0.8 mol) of stearic acid (trade name: Stearic Acid 90, manufactured by Miyoshi Oil & Fat Co., Ltd.). 8 mL of 10 w/v% aqueous sodium hydroxide solution as a catalyst was added thereto. Then, the esterification reaction was performed at 240°C in a nitrogen gas stream for about 2 hours until reaching an acid value of not more than 12. The resultant reaction mixture [Note1] was cooled down to about 180°C, the catalyst therein was neutralized by adding 1.84 g of phosphoric acid (85% by mass), the mixture was allowed to stand at the same temperature for about 1 hour, and about 40 g of the unreacted diglycerol which was separated was removed. Subsequently, the reaction mixture was cooled down to about 150°C, and 350 g of glycerol was added thereto. The mixture was mixed uniformly and allowed to stand for about 1 hour at the same temperature. About 324 g of the separated glycerol phase was removed by sucking with a Komagome pipette from above. The resultant polyglycerol fatty acid ester was distilled under reduced pressure at about 150°C and about 400 Pa to remove the remaining glycerol. Thus, about 295 g of diglycerol stearic acid ester (Trial Product 1) was obtained. The product showed an acid value of 1.6 and a hydroxy value of 270.

Note 1) The assumed content of unreacted polyglycerol based on random distribution rule was about 18% by mass.

Working Example 2

The molecular distilled residue obtained in Working Example 1 was subjected to molecular distillation again under high vacuum conditions of 240°C and 20 Pa to obtain about 1.5 kg of a distillate containing 1% by mass of glycerol, 4% by mass of diglycerol, 91% by mass of triglycerol, and 3% by mass of cyclic glycerol. Subsequently, 1% by mass of an activated carbon relative to the distillate was added thereto, thereby to discolor the distillate under reduced pressure. After filtration, the resultant triglycerol composition showed a hydroxy value of about 1170 and the average degree of polycondensation was about 3.0. To 1 L of a four necked flask comprising an agitator, a temperature gauge, a gas blowing tube, and a water separator were fed 240 g (about 1.0 mol) of the above triglycerol composition, 272 g (about 0.96 mol) of stearic acid (trade name: Stearic Acid 90, manufactured by Miyoshi Oil & Fat Co., Ltd.) . 10 mL of 10 w/v% aqueous sodium hydroxide solution as a catalyst was added thereto. Then, the esterification reaction was performed at 240°C in a nitrogen gas stream for about 2 hours until reaching an acid value of not more than 12. The obtained reaction mixture Note [2] was cooled down to about 180°C, the catalyst therein was neutralized by adding 2.3 g of phosphoric acid (85% by mass), the mixture was allowed to stand at the same temperature for about 1 hour, and about 40 g of the unreacted triglycerol which was separated was removed. Subsequently, the reaction mixture was cooled down to about 150°C and 420 g of glycerol was added thereto. The mixture was mixed uniformly and allowed to stand for about 1 hour at the same temperature. About 340 g of the separated glycerol phase was removed. The obtained polyglycerol fatty acid ester was distilled under reduced pressure at about 150°C and about 400 Pa to remove the remaining glycerol, and about 430 g of triglycerol stearic acid ester (Trial Product 2) was obtained. The product showed an acid value of 1.8 and a hydroxy value of 300.

Note 2) The assumed content of unreacted polyglycerol based on random distribution rule was about 17% by mass.

Working Example 3

20 kg of glycerol was fed to a reaction vessel comprising an agitator, a temperature gauge, a gas blowing tube, and a water separator. 200 mL of 20 w/v% aqueous sodium hydroxide solution as a catalyst was added thereto. Then, a polycondensation reaction of glycerol was performed at 250°C for 8 hours under nitrogen gas stream.

The obtained reaction product was cooled down to 90°C and about 40 g of phosphoric acid was added thereto for neutralization. Subsequently, 1% by mass of an activated carbon relative to the said neutralization reaction product was added thereto and the discolaration was performed under reduced pressure, followed by filtration. The resultant pentaglycerol composition showed a hydroxyl value of about 1011 and the average degree of polycondensation was about 5.0.

To 2 L of a four necked flask comprising an agitator, a temperature gauge, a gas blowing tube, and a water separator were fed 388 g (about 1.0 mol) of the above pentaglycerol composition, 348 g (about 1.23 mol) of stearic acid (trade name: stearic acid 90, manufactured by Miyoshi Oil & Fat Co. , Ltd.). 15 mL of 10 w/v% aqueous sodium hydroxide solution as a catalyst was added thereto. Then, the esterification reaction was performed at 240°C in a nitrogen gas stream for about 2 hours until reaching an acid value of not more than 12. The obtained reaction mixture Note [3] was cooled down to about 180ºC, the catalyst therein was neutralized by adding 3.5 g of phosphoric acid (85% by mass), and the mixture was allowed to stand at the same temperature for about 1 hour. Subsequently, the reaction mixture was cooled down to about 150°C, 640 g of glycerol was added thereto and mixed uniformly. The mixture was allowed to stand at the same temperature for about 1 hour, and about 430 g of the glycerol phase which was separated was removed. The obtained polyglycerol fatty acid ester was distilled under reduced pressure under conditions of about 150°C and about 400 Pa to remove the remaining glycerol, thereby to obtain about 641 g of pentaglycerol stearic acid ester (Trial Product 3). The product showed an acid value of 2. 4 and a hydroxy value of 359.

Note 3) The assumed content of unreacted polyglycerol based on random distribution rule was about 15% by mass.

Working Example 4

20 kg of glycerol was fed to a reaction vessel comprising an agitator, a temperature gauge, a gas blowing tube, and a water separator. 200 mL of 20 w/v% aqueous sodium hydroxide solution as a catalyst was added thereto. Then, a polycondensation reaction of glycerol was performed at 250°C for 10 hours in a nitrogen gas stream.

The obtained reaction product was cooled down to 90°C and about 40 g of phosphoric acid was added thereto for neutralization. Subsequently, 1% by mass of an activated carbon relative to the said neutralization reaction product was added thereto and the discolaration was performed under reduced pressure, followed by filtration. The obtained octaglycerol composition showed a hydroxy value of about 920 and the average degree of polycondensation was about 8.0.

To 2 L of a four necked flask comprising an agitator, a temperature gauge, a gas blowing tube, and a water separator were fed 610 g (about 1.0 mol) of the above octaglycerol composition, 405 g (about 1.43 mol) of a stearic acid (trade name: Stearic Acid 90, manufactured by Miyoshi Oil & Fat Co., Ltd.). 20 mL of 10 w/v% aqueous sodium hydroxide solution as a catalyst was added thereto. Then, the esterification reaction was performed at 240°C in a nitrogen gas stream for about 2 hours until reaching an acid value of not more than 12. The obtained reaction mixture Note [4]) was cooled down to about 180°C, the catalyst therein was neutralized by adding 4.6 g of phosphoric acid (85% by mass), and the mixture was allowed to stand at the same temperature for about 1 hour. Subsequently, the reaction mixture was cooled down to about 150°C, 915 g of glycerol was added thereto, the mixture was mixed uniformly and allowed to stand for about 1 hour at the same temperature, and about 342 g of the glycerol phase which was separated was removed. The obtained polyglycerol fatty acid ester was distilled under reduced pressure at about 150°C and about 400 Pa to remove the remaining glycerol, thereby to obtain about 1278 g of octaglycerol stearic acid ester (Trial Product 4). The product showed an acid value of 2.8 and a hydroxy value of 416. Note 4) The assumed content of unreacted polyglycerol based on random distribution rule was about 14% by mass.

Test Example 1

Free polyglycerol contents (containing glycerol) in the polyglycerol fatty acid esters (Trial Products 1 to 4) obtained in Working Examples 1 to 4 were determined. The results are shown in Table 1.

[Quantitative method]

A glass column (length: 21 cm, diameter: 2 cm) was packed with about 30 g of reversed phase silica gel (trade name: Inertsil ODS-3; manufactured by GL Sciences, Inc.) by dry process. About 10 g of a sample weighed precisely was dissolved in 50 mL of 25 v/v% aqueous methanol solution. Subsequently, this solution was flowed into the upper part of the column, and 200 mL of 25 v/v% aqueous methanol solution was passed through the column at a flow rate of 1 mL/min to collect an eluate. The eluate was poured into a concentration flask with predetermined weight, and concentrated under conditions of about 90°C and about 4 kPa with a rotary evaporator, and then allowed to stand for cooling in a desiccator. Then, the total weight was measured precisely to determine free polyglycerol content (% by mass) by the following equation.

$$\text{Free Polyglycerol content} = \frac{\text{Total weight(g)} - \text{Flask weigh (g)}}{\text{Sample weight (g)}} \times 100 (\% \text{ by mass})$$

Table 1

|  | Free polyglycerol content (% by mass) |
|---|---|
| Trial Product 1 | 3.1 |

(continued)

|  | Free polyglycerol content (% by mass) |
| --- | --- |
| Trial Product 2 | 3.6 |
| Trial Product 3 | 4.4 |
| Trial Product 4 | 4.7 |

Test Example 2

The polyglycerol fatty acid esters (Trial Products 1 to 4) obtained in Working Examples 1 to 4 were spray-cooled to observe the characteristics of the obtained powder particles. The results are shown in Table 2.

[Operation procedure]

A spray dryer equipment for research and development (Type: L-8i; manufactured by Ohkawara Kakoki Co., Ltd.) was used. About 200 g of each sample was melted by heating to about 80°C. The melted liquid was sprayed from a pressurized nozzle into a chamber wherein the inside temperature of the chamber was adjusted to about 5 to 10°C by injecting liquid nitrogen thereto, resulting in recovering the cooled and solidified powder particles at the bottom of the chamber.

Table 2

|  | Characteristics of powder particles |
| --- | --- |
| Trial Product 1 | Pulverization is possible. |
| Trial Product 2 | Pulverization is possible. Smooth, Very good powder |
| Trial Product 3 | Pulverization is possible. Smooth, Good powder |
| Trial Product 4 | Pulverization is possible. Smooth, Good powder |

Any Trial Products 1 to 4 were found to be a good powder. Since the same kind of any polyglycerol fatty acid esters produced by the conventional method are very sticky and difficult to be pulverized, the effect of the present invention is apparent when compared to the conventional products.

**Claims**

1. A process for producing a polyglycerol fatty acid ester, which comprises adding glycerol to a reaction mixture obtained by a direct esterification reaction between polyglycerol and a fatty acid at the temperature from 60°C to less than 180°C, and separating and removing the glycerol phase containing unreacted polyglycerols.

**Patentansprüche**

1. Verfahren zur Herstellung eines Polyglycerinfettsäureesters, das die Zugabe von Glycerin zu einer Reaktionsmischung, die durch direkte Veresterungsreaktion von Polyglycerin und einer Fettsäure bei einer Temperatur von 60°C bis weniger als 180°C erhalten wurde, und die Abtrennung und Entfernung der Glycerinphase, die nicht umgesetztes Polyglycerin enthält, umfasst.

**Revendications**

1. Procédé de production d'un ester polyglycérolique d'acide gras, dans lequel du glycérol est ajouté à un mélange réactionnel obtenu par une réaction d'estérification directe entre du polyglycérol et un acide gras à une température de 60°C à moins de 180°C, et la phase glycérolique contenant des polyglycérols non réagis est séparée et éliminée.